# EUROPEAN PATENT APPLICATION

(11) **EP 3 133 397 A1**
(43) Date of publication of application: **22.02.2017**
(21) Application number: 16184911.2
(22) Date of filing: 19.08.2016
(51) Int. Cl.: G01N 33/50, A01N 1/02, C12N 5/00

(54) **SPERM COUNT QUALITY CONTROL**

(30) Priority: 19.08.2015 US 201562206919 P
(71) Applicant: Streck, Inc., Lavista, NE 68128 (US)
(72) Inventor: HUNSLEY, Bradford, Papillion, NE 68133 (US); HORSTMAN, Cathy, Omaha, NE 68144 (US); MCCARTHY, Kitty, Omaha, NE 68130 (US)
(74) Representative: Bawden, Peter Charles

(57) **Abstract**

A reference control composition for use therein and methods for making the same, including a plurality of spermatozoa from a non-human source for simulating human spermatozoa and an effective amount of an agent for maintaining the plurality of spermatozoa in a substantially aggregate-free condition.

## Description

### FIELD OF THE INVENTION

This invention relates to a control composition for simulating human spermatozoa and more particularly to a simulated human spermatozoa for use for controls for helping assure quality of semen analysis.

### BACKGROUND

Semen analysis evaluates certain characteristics of semen and the sperm contained therein. Semen analysis may provide information related to sperm count, sperm motility and sperm morphology. Laboratory semen analysis may be employed as part of a pre-pregnancy test, an infertility investigation, a sperm donor test or confirmation of a successful vasectomy. A popular tool for the analysis of semen is a hemocytometer. Hemocytometers typically employ a cover glass, a cover glass support and a counting chamber or chambers of a known volume, being adapted to receive one or more semen dilutions. A hemocytometer may be used to count the number of spermatozoa in a specific volume of a fluid, or to determine spermatozoa concentration.

It is regarded as important to employ quality control measures during sperm testing. According to the Clinical Laboratory Improvement Amendments (CLIA), quantitative semen analysis is a high complexity test and requires two levels of quality controls on each day of patient testing. Post-vasectomy screening, which tests for the presence or absence of sperm, is a moderately complex test and requires a positive and negative control each day of testing. In addition, the CLIA require semen analysis proficiency testing for analysts. In order to function as an analytical process quality control measure for clinical laboratory applications, controls are typically employed to simulate one or more detectable characteristics of a sample for analysis by a clinical laboratory instrument. For example, when a microscope is employed, it may be important for a control material to visibly resemble a significant feature (e.g., size, morphology, etc.) of the intended sample.

Beads, spheres and like particles have been used as a sperm count quality control. See US 2007/0243578 A1 incorporated by reference herein. One such bead sperm count control has been made by Hamilton Thorpe (Beverly, Massachusetts), under the designation Accu-beads™. However, the beads, spheres and like particles do not resemble sperm.

Stabilized human sperm has been employed as a sperm control. One such human sperm count control has been made by Fertility Solutions (Cleveland, Ohio), under the designation AQC™. Such controls are based upon sperm collected and stabilized from human donors.

However, though such control compositions have been employed reliably for many years, there remains a need for continued improvement to sperm controls. For example, concerns (whether justifiable or not) about the potential for human-to-human transmission of infectious agents during handling of sperm controls have helped to drive a need for sperm analogs from non-human sources. Financial concerns, as sourcing from human donors may also entail screening for disease states and payment to donors, has also played a role in the drive for non-human sourcing.

Furthermore, due to a combination of size, shape, and surface characteristics of sperm, and potential for variation of semen characteristics from human donor to donor, there is an ongoing potential that sperm aggregates will form and render a control unsatisfactory. Sperm aggregates may adhere to the analytic instrument used for semen analysis. The World Health Organization Laboratory Manual for the Examination and Processing of Human Semen, incorporated by reference herein, defines aggregation of spermatozoa as "[t]he adherence of either immotile spermatozoa to each other or of motile spermatozoa to mucus strands, non-sperm cells or debris..." and defines agglutination as "motile spermatozoa sticking to each other, head-to-head, tail-to-tail or in a mixed way." The potential for aggregation of spermatozoa in a sperm control composition, when subjected to analysis, may lead to inaccurate sperm analysis results.

Hence, as the above illustrates, there is an ongoing need for an improved sperm control composition that can meet some or all of the above needs. For example, there is a need for a sperm control that employs spermatozoa from a non-human source. There is a need for a sperm control that has a relatively long useful life. There is a need for a sperm control that avoids aggregate formation during its useful life. There is a need for a sperm control composition that has one or more detectable characteristics (e.g., size, shape, etc.) resembling that of human sperm. There is also a need for a sperm control composition that is relatively safe and/or easy to make, handle and/or use.

The present invention addresses one or more of the above needs by providing a non-human sperm control composition and a method for making the non-human sperm control composition.

### SUMMARY

The present teachings contemplate a reference control composition comprising a plurality of spermatozoa from a non-human source for simulating human spermatozoa and an effective amount of an agent for maintaining the plurality of spermatozoa in a substantially aggregate-free condition.

The plurality of spermatozoa may have a shape substantially resembling human spermatozoa so that the reference control composition can be used for quality control purposes to test the function of a device for measuring predetermined values for human spermatozoa. The plurality of spermatozoa may be present in a predetermined concentration. The predetermined concentration may be in an amount corresponding to a low level of human sperm, a normal level of human sperm or a high level of human sperm.

The non-human source may be a mammal. The non-human source may be selected from at least one of porcine, equine, bovine, caprine or ovine. The non-human source may be selected from one or more of boar, ram, stallion or bull.

The concentration of the agent for maintaining the spermatozoa in a substantially aggregate-free condition may be from about 0.01% to about 2.5%. The agent may include one or more ingredients having one or any combination of the following: a pH of about 7 to about 9; a hydrophilic-lipophilic balance (HLB) value of about 15.5 to about 18.5; a viscosity of about 0.5 Pa*s (500 cP) to about 0.55 Pa*s (550 cP) at 25°C; a density of about 1.075 g/mL to 1.100 g/mL at 25°C; and a surface tension of about 39 dynes/cm to about 52 dynes/cm at 25°C. The agent may include one or more ingredients having one or any combination of the following: a pH of about 5 to about 7.5; a hydrophilic-lipophilic balance (HLB) value of about 24 to about 28; a viscosity of about 1 Pa*s (1000 cP) to about 2.3 Pa*s (2300 cP) at 77°C; and a surface tension of about 48 dynes/cm to about 50 dynes/cm at 25°C. The agent may include one or more ingredients having one or any combination of the following: a pH of about 5 to about 9; a hydrophilic-lipophilic balance (HLB) value of about 15.5 to about 28; and a surface tension of about 39 dynes/cm to about 52 dynes/cm at 25°C.

The agent may include one or more octylphenol ethoxylates. The agent may include one or more block copolymers. The agent may include at least one hydrocarbon chain with one or more ether groups. The agent may include one or more alcohol groups. The agent may include a molecule that has a hydrophilic portion and a hydrophobic portion. The agent may include an alkyl ester and/or an alkoxylated ester. The agent may include an alkyl ether and/or an alkoxylated ether. The agent may include nonoxynol. The agent may include alkylphenol ether. The agent may include a plurality of repeating alkylene oxide units.

The pH of the control composition may be from about 7 to about 9.5. The control composition may have an open vial stability of at least about six weeks at a temperature of about 2°C - 10°C. The control composition may have a closed vial stability of at least about 12 months at a temperature of about 2°C - 10°C. The control composition may have a closed vial stability of at least about 2 years at a temperature of about 2°C - 10°C.

The present teachings further contemplate a method for preparing a reference control composition comprising obtaining non-human semen containing a plurality of spermatozoa that has been contacted within about thirty minutes of collection with phosphate buffered saline and at least one enzyme and adding to the contacted semen a diluent composition comprising one or any combination of a buffer, a viscosity agent, a fixative agent or organic biocide agent and at least one agent for maintaining the plurality of spermatozoa in a substantially aggregate-free condition. The diluent composition may be added within four hours of semen collection.

The one or more of the plurality of spermatozoa of the non-human semen may have morphological attributes of human semen specimens in that the spermatozoa have substantially oval shaped heads and a tail that is either straight, coiled or overlapped with other adjacent spermatozoa. The non-human semen may be of boar origin. The amount of non-human semen obtained may be from greater than about 0.1x10⁶mL to less than about 100x106/mL.

The amount of the at least one agent for maintaining the plurality of spermatozoa in a substantially aggregate-free condition may be from about 0.01% to 2.5%. The enzyme may be selected from a group consisting of: chymotrypsin, protein kinase K, trypsin, phenylmethanesulfonyl fluoride (PMSF), and any combination thereof. The viscosity agent may be selected from a group consisting of: polyvinylpyrrolidone, polyethylene glycol (PEG), hydroxyethylcellulose (HEC), Hydroxymethylcellulose (HMC), xanthan gum, alginic acid, gelatin, and any combination thereof. The fixative agent or biocide agent may be selected from a group consisting of aldehyde, oxazolidine, alcohol, cyclic urea and mixtures thereof. The method may further include using an analytic instrument.

The present teachings provide a reference control composition from a non-human source that accurately mimics the morphological attributes of human spermatozoa. The present teachings provide a reference control composition in which the plurality of spermatozoa are in a substantially aggregate-free condition. The present teachings provide a sperm control composition that is relatively safe and/or easy to make, handle and/or use. The present teachings provide a reference control composition which has a relatively long useful life.

### BRIEF DESCRIPTION OF THE DRAWINGS

FIG. 1 is a depiction of a species comparison of spermatozoa.
FIG. 2 is a microscopic photograph on a hemocytometer that illustrates the stability of the sperm reference control composition at 87 days at a temperature of 6°C.
FIG. 3 is a microscopic photograph on a hemocytometer that illustrates the stability of the sperm reference control composition at 48 days at a temperature of 50°C.

### DETAILED DESCRIPTION

The explanations and illustrations presented herein are intended to acquaint others skilled in the art with the teachings, its principles, and its practical application. Those skilled in the art may adapt and apply the teachings in its numerous forms, as may be best suited to the requirements of a particular use. Accordingly, the specific embodiments of the present teachings as set forth are not intended as being exhaustive or limiting of the teachings. The scope of the teachings should, therefore, be determined not with reference to the above description, but should instead be determined with reference to the appended claims, along with the full scope of equivalents to which such claims are entitled. The disclosures of all articles and references, including patent applications and publications, are incorporated by reference for all purposes. Other combinations are also possible as will be gleaned from the following claims, which are also hereby incorporated by reference into this written description.

The present teachings provide a reference control composition comprising a plurality of spermatozoa from a non-human source for simulating human spermatozoa and an effective amount of an agent for maintaining the plurality of spermatozoa in a substantially aggregate-free condition. The present teachings provide a method for preparing a reference control composition comprising obtaining non-human semen containing a plurality of spermatozoa that has been contacted within about thirty minutes of collection with phosphate buffered saline and at least one enzyme and adding to the contacted semen a diluent composition comprising one or any combination of a buffer, a viscosity agent, a fixative agent or organic biocide agent and at least one agent for maintaining the plurality of spermatozoa in a substantially aggregate-free condition.

The reference control composition includes a plurality of spermatozoa from a non-human source for simulating human spermatozoa and effective amount of an agent for maintaining the plurality of spermatozoa in a substantially aggregate-free condition. At least 95% of the volume of the reference control composition may be in an aggregate-free condition. At least 90% of the volume of reference control composition may be in an aggregate-free condition.

The plurality of spermatozoa may have a shape substantially resembling human spermatozoa so that the reference control composition can be used for quality control purposes to test the function of a device for measuring predetermined values for human spermatozoa. The reference control composition may be used for quality control purposes to confirm the accuracy of counting procedures for semen analysis. The reference control composition may be may be used for quality control purposes to test for the presence or absence of sperm in post-vasectomy semen analysis. The reference control composition may be used for quality control purposes to test the proficiency of clinical analysts in sperm count testing.

The plurality of spermatozoa may be present in a predetermined concentration. The predetermined concentration may be an amount corresponding to a low level of human sperm, a normal level of human sperm or a high level of human sperm. The predetermined concentration may contain no sperm.

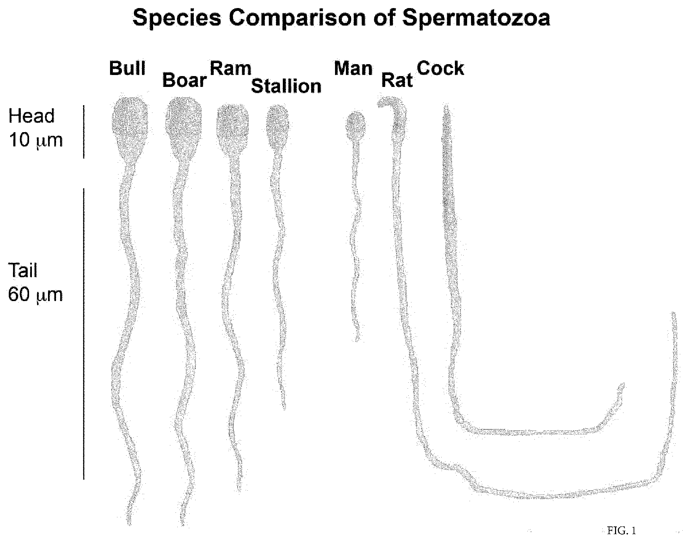

The non-human source may be a mammal. Table 1 below illustrates the testicular characteristics and sperm production estimates of some sexually mature non-human mammals. The non-human source may be selected from at least one of porcine, equine, bovine, caprine or ovine or any combination thereof. The non-human source may be selected from one or more of boar, ram, stallion or bull. FIG 1. depicts a species comparison of spermatozoa and serves to illustrate the attributes of several non-human sources that closely align with human sperm (i.e. boar, bull, ram, stallion). Though not necessary in every instance, desirably, the source will provide spermatozoa that have a one or more characteristics that (especially upon treatment) will substantial mimic a characteristic of human spermatozoa. For example, the non-human source spermatozoa may have a substantially similar morphology as a human spermatozoa (e.g., it has a bulbous head and a relatively narrow tail as compared with the bulbous head, each being positioned generally as with a human spermatozoa). The spermatozoa may have substantially similar dimensions as a human spermatozoa (e.g., it has a head and a tail, each being dimensionally proportional to that of a human spermatozoa for example, with dimensions differing from those of a human spermatozoa by less than about 50%, 35%, or 25%). The spermatozoa for use in the control from a non-human source may be from only a porcine source, and/or from only one of the aforementioned sources.

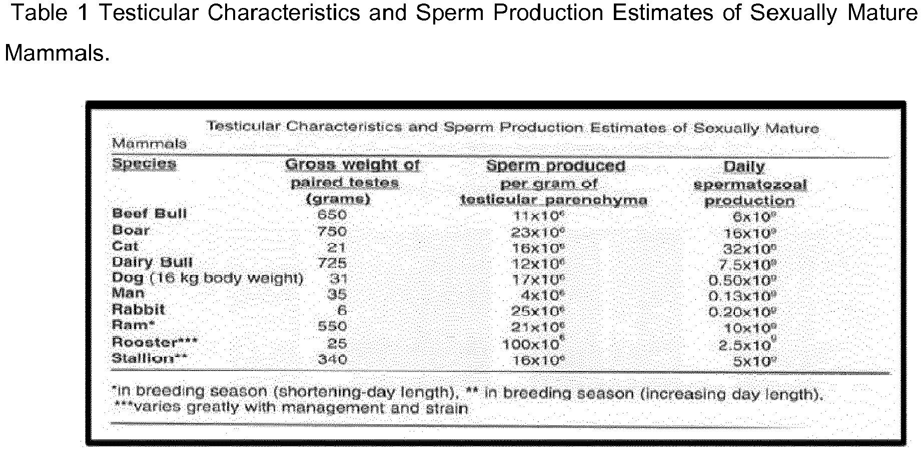

The reference control composition may include a preserved plurality of non-human spermatozoa, wherein each of the plurality of spermatozoa are at least about 1 to about 3 times the size of a human spermatozoa. The reference control composition may include preserved plurality of non-human spermatozoa, wherein a head portion of each of the plurality of spermatozoa are at least about 25 µM to about 40µM at their largest diameter.

The reference control composition includes an effective amount of an agent for maintaining the plurality of spermatozoa in a substantially aggregate-free condition. The concentration of the agent may be from about 0.01% to about 2.5%.

The agent may include one or more ingredients having one or any combination of the following: a pH of about 7 to about 9; a hydrophilic-lipophilic balance (HLB) value of about 15.5 to about 18.5; a viscosity of about 0.5 Pa*s (500 cP) to about 0.55 Pa*s (550 cP) at 25°C; a density of about 1.075 g/mL to 1.100 g/mL at 25°C; and a surface tension of about 39 dynes/cm to about 52 dynes/cm at 25°C. The agent may include one or more ingredients having one or any combination of the following: a pH of about 5 to about 7.5; a hydrophilic-lipophilic balance (HLB) value of about 24 to about 28; a viscosity of about 1 Pa*s (1000 cP) to about 2.3 Pa*s (2300 cP) at 77°C; and a surface tension of about 48 dynes/cm to about 50 dynes/cm at 25°C. The agent may include one or more ingredients having one or any combination of the following: a pH of about 5 to about 9; a hydrophilic-lipophilic balance (HLB) value of about 15.5 to about 28; and a surface tension of about 39 dynes/cm to about 52 dynes/cm at 25°C.

The agent includes at least one hydrocarbon chain. The hydrocarbon chain may include a branched portion, a linear portion and/or an aromatic portion. The hydrocarbon chain may include one or more ether groups (e.g., one or more polyether groups). The agent may include a molecule that has a hydrophilic portion (e.g., a head) and a hydrophobic portion (e.g., a tail). The hydrophilic portion may have a positive net charge, a negative net charge or a combination of both. The hydrophilic portion may have a neutral net charge. The agent may include one or more alcohol groups. The agent may include an alkyl ester and/or an alkoxylated ester. The agent may include an alkyl ether and/or an alkoxylated ether. The agent may include a polyether compound. The agent may include a nonoxynol. The agent may be include alkylphenol ether. The agent may include one or more octylphenol ethoxylates. The agent may include one or more block copolymers. The agent may include a multi-block copolymer. The agent may include a poloxamer. The agent may include a plurality of repeating alkylene oxide units. The repeating alkylene oxide units may be present from about 1 to about 120 units per molecule, about 3 to about 90 units per molecule, or about 5 to about 105 units per molecule. The repeating alkylene units may be present from about 8 to about 90 units per molecule, or from about 12 to about 75 units per molecule (such as about 25, 35, 45, 55 or about 65 units per molecule). For example, the agent may include a plurality of ethylene oxide units, a plurality of propylene oxide units, or both. The agent may include benzalkonium chloride (BAC).

The pH of the control composition may be such so as not to significantly change the normal pH range of the semen. The pH of the control composition may be from about 7 to about 9.5. The pH of the control composition may be from about 6.5 to about 9. The pH of the control composition may be from about 7.0 to about 8.0.

The control composition should have a relatively long useful life (e.g., a lengthy shelf life). The control composition may have an open vial stability of at least about four weeks at a temperature of about 2°C - 10°C. The control composition may have an open vial stability of at least about six weeks at a temperature of about 2°C - 10°C. The control composition may have a closed vial stability of at least about 10 months at a temperature of about 2°C - 10°C. The control composition may have a closed vial stability of at least about 12 months at a temperature of about 2°C - 10°C. The control composition may have a closed vial stability of at least about 2 years at a temperature of about 2°C - 10°C.

The method for preparing a reference control composition may include obtaining non-human semen containing a plurality of spermatozoa that has been contacted within about thirty minutes of collection with phosphate buffered saline and at least one enzyme. The method may further include adding to the contacted semen a diluent composition comprising one or any combination of a buffer, a viscosity agent, a fixative agent or organic biocide agent and at least one agent for maintaining the plurality of spermatozoa in a substantially aggregate-free condition.

The diluent composition may be added within four hours of semen collection. The method may further include storing the tube with resulting diluent mixture in an upright position for about 3-7 days at room temperature. The method may further include aspirating fluid from the diluent mixture in the tube and vortexing cells for about 60 seconds. The method may further include re-suspending the cells by adding about 100 ml of additional diluent composition to the tube. The method may further include twice repeating aspirating fluid from the diluent mixture in the tube and vortexing cells for about 60 seconds and re-suspending the cells by adding about 100 ml of additional diluent composition to the tube. The method may further include preparing a 1:500 dilution and analyzing sperm count and morphology using an analytic instrument. The method may further include diluting the sample to about 45.0x10⁶/ml. The method may further include confirming sperm count by preparing a 1:200 dilution and analyzing sperm count and morphology using an analytic instrument. The method may further include diluting the diluent mixture with diluent composition.

The one or more of the plurality of spermatozoa of the non-human semen may have morphological attributes of human semen specimens in that the spermatozoa have substantially oval shaped heads and a tail that is either straight, coiled or overlapped with other adjacent spermatozoa. The non-human semen may be of boar origin. The amount of non-human semen collected may be from greater than about 0.1x10⁶mL to less than about 100x10⁶/mL.

**Table 2 depicts the average number of sperm per ejaculate (in millions) for man and various animals.**

| Animal | Average number of sperm per ejaculate (in millions) |
|---|---|
| Mouse | 50 |
| Rat | 58 |
| Guinea | 80 |
| Pig | 280 |
| Rabbit | 280 |
| Man | 1000 |
| Sheep | 3000 |
| Cow | 8000 |
| Pig | |

The reference control composition contains at least one agent for maintaining the plurality of spermatozoa in a substantially aggregate-free condition. The agent may lower the surface tension. The amount of the at least one agent for maintaining the plurality of spermatozoa in a substantially aggregate-free condition may be from about 0.01% to 2.5%. The at least one agent for maintaining the plurality of spermatozoa in a substantially aggregate-free condition may be about 0.01% to about 2.50% of the reference control composition. The at least one agent for maintaining the plurality of spermatozoa in a substantially aggregate-free condition may be about 0.01% to about 5.0% of the reference control composition.

The non-human semen containing plurality of spermatozoa may be contacted within about thirty minutes of collection with a buffer. The buffer solution may keep its pH at a nearly constant value. The buffer may be an organic buffer. The buffer may be an inorganic buffer. The buffer may be 4-2-hydroxyethyl-1-piperazineethanesulfonic acid (HEPES), tris(hydroxymethyl)methylamine (TRIS), or 3-(N-morpholino)propanesulfonic acid (MOPS). The buffer may include citrate. The buffer may include phosphate. The buffer may include bicarbonate. The buffer may be sodium bicarbonate. The buffer may be about 0.50% to about 15.0% of the reference control composition.

The non-human semen containing a plurality of spermatozoa may be contacted within about thirty minutes of collection with at least one enzyme. Enzymes may perform proteolysis, the breakdown of proteins into smaller polypeptides or amino acids. The enzyme may breakdown proteins and polypeptides present in seminal fluid. The enzyme may be a digestive enzyme. The enzyme may be chymotrypsin, protein kinase K, trypsin, phenylmethanesulfonyl fluoride (PMSF), and any combination thereof. The enzyme may about 0.10% to about 10.0% of the reference control composition.

The diluent composition may include a viscosity modification agent. The diluent composition may include a combination of viscosity modification agents. The viscosity modification agent may increase the viscosity of a liquid. The viscosity modification agent may improve the suspension of other ingredients or emulsions. The viscosity modification agent may be a gelling agent. The viscosity modification agent may be thixotropic agent. The viscosity modification agent may be a polymer. The viscosity modification agent may be an oligomer. The viscosity modification agent may be a polysaccharide. The viscosity modification agent may be a starch. The viscosity modification agent may be a vegetable gum. The viscosity modification agent may be a protein. The viscosity modification agent may be a chemically substituted cellulose macromolecule. The viscosity modification agent may be polyvinylpyrrolidone, polyethylene glycol (PEG), hydroxyethylcellulose (HEC), Hydroxymethylcellulose (HMC), xanthan gum, alginic acid, gelatin, and any combination thereof. The viscosity modification agent may be about 0.01% to about 1.00 % of the reference control composition. The viscosity modification agent may be about 0.01% to about 3.00% of the reference control composition. The viscosity modification agent may be about 0.01% to about 5.00% of the reference control composition.

The diluent composition may include a fixative agent and/or biocide agent. The diluent composition may include a combination of fixative agents and/or biocide agents. The fixative agent may stabilize the structural integrity of the sperm. The fixative agent may include formaldehyde, glutaraldehyde, formalin, diazolidinyl urea (DU) or imidazolidinyl urea (IDU). The biocide agent may destroy, render harmless, prevent the action of, or otherwise exert a controlling effect on any harmful organism by chemical or biological means. The biocide agent may be a preservative. The fixative agent or biocide agent may be an aldehyde, oxazolidine, alcohol, cyclic urea and mixtures thereof. The at least one fixative agent and/or biocide agent may be about 3% to about 10.0% of the reference control composition. The at least one fixative agent and/or biocide agent may about 5.0% to about 15.0% of the reference control composition.

The method may include analyzing sperm count and morphology using an analytic instrument. The analytic instrument may be a hemocytometer, spermocytometer, sperm counting chamber, sperm counting device, microscope tally device, or computer assisted sperm analyzer.

### EXAMPLE 1

Fresh boar semen is processed as follows to prepare a stabilized non-human sperm control. Combine fresh boar ejaculate at a 1:1 ratio in phosphate buffered saline (PBS) plus 5% Chymotrypsin (CAS Registry #9004-07-3) with a pH of 7.0 ± 0.05. The 1:1 ratio is defined as 20 ml of boar ejaculate to 20 ml of phosphate buffered saline (PBS) plus 5% Chymotrypsin in a 50 ml conical tube. Invert 10 times. One inversion is a complete turn of the wrist, 180 degrees and back. Add diluent composition at a ratio of 1:1 within 4 hours of semen collection. The diluent composition includes buffer, viscosity modification agent(s), fixative agent(s) and/or biocide agent(s) and at least one agent for maintaining the plurality of spermatozoa in a substantially aggregate-free condition Gravity settle 3-7 days at room temperature. Aspirate, vortex for 60 seconds, re-suspend with additional 100ml of diluent composition and repeat two times. Prepare a 1:500 dilution and count using a hemocytometer. Report the number of heads and sperm with twisted tails. Percentage of intact sperm with good morphology must be greater than 75%. After confirming morphology of the sperm, dilute to approximately 45.0x10⁶/ml. Confirm sperm count by making a 1:200 dilution and counting on a hemocytometer. Dilute with diluent composition to produce lower levels (i.e. 2.0x10⁶/ml & 5.0x10⁶/ml).

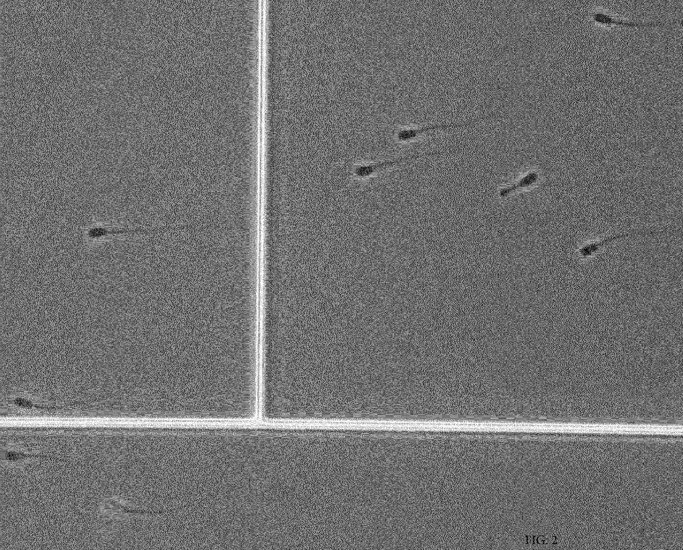

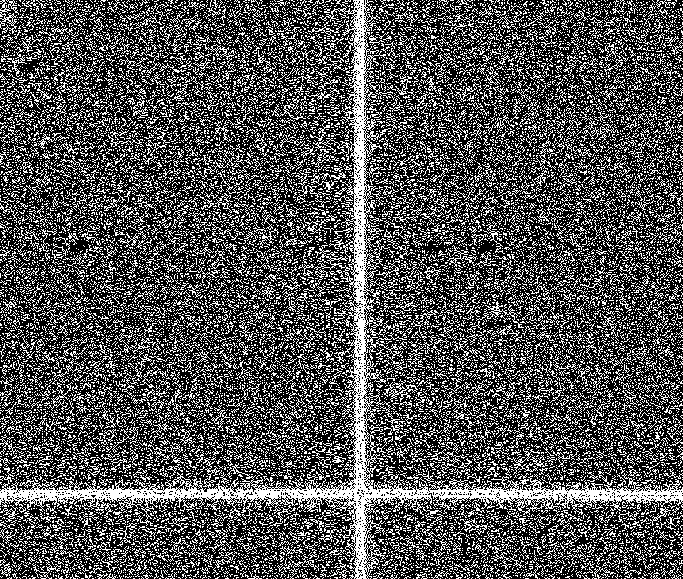

FIG 2. illustrates the effective preservation of the morphological attributes of the stabilized sperm at 87 days at a refrigerated temperature of 6°C. FIG. 3 illustrates the effective preservation of the morphological attributes of the stabilized sperm at 48 days under stressed thermal conditions of 50°C. Using an elevated temperature extrapolation model, 48 days of real-time stability at 50°C serves to predict approximately 2 years of product shelf-life at 6°C.

The results illustrate consistent quantification of the total sperm count. Table 3 depicts the stability data collected at varying time intervals at a refrigerated temperature of 6°C. Table 4 depicts the stability data collected at varying time intervals at an elevated temperature of 50°C.

**Table 3 Stability at 6°C**

| Hemocytometer (M/ml) 6° C | | | | | | |
|---|---|---|---|---|---|---|
| Day | Level 1 Count | Level 1 Twists % | Level 2 Count | Level 2 Twists % | Post-Vas Positive Count | Post-Vas Positive Twists % |
| 0 | -- | -- | -- | -- | -- | -- |
| 12 | 4.2 | 7.8 | 48.3 | 2.1 | 2.2 | 3.5 |
| 26 | 4.7 | 3.7 | 51.3 | 5.4 | 2.2 | 6.9 |
| 48 | 5.1 | 3.9 | 48.3 | 4.2 | 2.3 | 4.4 |
| 66 | 4.9 | 4.1 | 46.0 | 3.2 | 2.3 | 3.2 |
| 87 | 5.3 | 5.2 | 50.8 | 7.8 | 2.1 | 5.9 |

**Table 4 Stability at 50°C**

| Hemocytometer (M/ml) 50° C | | | | | | |
|---|---|---|---|---|---|---|
| Day | Level 1 Count | Level 1 Twist % | Level 2 Count | Level 2 Twist % | Post-Vas Positive Count | Post-Vas Positive Twist % |
| 0 | -- | -- | -- | -- | -- | -- |
| 5 | 4.8 | 6.8 | 47.3 | 6.4 | 2.2 | 4.6 |
| 12 | 5.3 | 4.3 | 41.3 | 6.1 | 2.2 | 1.7 |
| 18 | 5.8 | 5.6 | 48.0 | 6.3 | 2.1 | 7.3 |
| 24 | 5.2 | 4.9 | 47.8 | 6.8 | 2.3 | 7.2 |
| 31 | 5.6 | 4.5 | 49.3 | 6.1 | 2.5 | 6.6 |
| 39 | 5.4 | 4.2 | 48.0 | 4.2 | 2.5 | 4.5 |
| 48 | 5.4 | 4.7 | 48.5 | 6.7 | 2.5 | 7.5 |

Any numerical values recited herein include all values from the lower value to the upper value in increments of one unit provided that there is a separation of at least 2 units between any lower value and any higher value. As an example, if it is stated that the amount of a component or a value of a process variable such as, for example, temperature, pressure, time and the like is, for example, from 1 to 90, preferably from 20 to 80, more preferably from 30 to 70, it is intended that values such as 15 to 85, 22 to 68, 43 to 51, 30 to 32 etc. are expressly enumerated in this specification. For values which are less than one, one unit is considered to be 0.0001, 0.001, 0.01 or 0.1 as appropriate. These are only examples of what is specifically intended and all possible combinations of numerical values between the lowest value and the highest value enumerated are to be considered to be expressly stated in this application in a similar manner.

Unless otherwise stated, all ranges include both endpoints and all numbers between the endpoints. The use of "about" or "approximately" in connection with a range applies to both ends of the range. Thus, "about 20 to 30" is intended to cover "about 20 to about 30", inclusive of at least the specified endpoints.

The disclosures of all articles and references, including patent applications and publications, are incorporated by reference for all purposes. The term "consisting essentially of" to describe a combination shall include the elements, ingredients, components or steps identified, and such other elements ingredients, components or steps that do not materially affect the basic and novel characteristics of the combination. The use of the terms "comprising" or "including" to describe combinations of elements, ingredients, components or steps herein also contemplates embodiments that consist essentially of or even consists of the elements, ingredients, components or steps. Plural elements, ingredients, components or steps can be provided by a single integrated element, ingredient, component or step. Alternatively, a single integrated element, ingredient, component or step might be divided into separate plural elements, ingredients, components or steps. The disclosure of "a" or "one" to describe an element, ingredient, component or step is not intended to foreclose additional elements, ingredients, components or steps.

It is understood that the above description is intended to be illustrative and not restrictive. Many embodiments as well as many applications besides the examples provided will be apparent to those of skill in the art upon reading the above description. The scope of the invention should, therefore, be determined not with reference to the above description, but should instead be determined with reference to the appended claims, along with the full scope of equivalents to which such claims are entitled. The disclosures of all articles and references, including patent applications and publications, are incorporated by reference for all purposes. The omission in the following claims of any aspect of subject matter that is disclosed herein is not a disclaimer of such subject matter, nor should it be regarded that the inventors did not consider such subject matter to be part of the disclosed inventive subject matter.

## Claims

1. A reference control composition comprising:
a. a plurality of spermatozoa from a non-human source for simulating human spermatozoa; and
b. an effective amount of an agent for maintaining the plurality of spermatozoa in a substantially aggregate-free condition.

2. The reference control composition of claim 1, wherein plurality of spermatozoa have a shape substantially resembling human spermatozoa so that the reference control composition can be used for quality control purposes to test the function of a device for measuring predetermined values for human spermatozoa.

3. The reference control composition of claim 1 or claim 2, wherein the plurality of spermatozoa are present in a predetermined concentration.

4. The reference control composition of any of the preceding claims, wherein the predetermined concentration is in an amount corresponding to a low level of human sperm, a normal level of human sperm or a high level of human sperm.

5. The reference control composition of any of the preceding claims, wherein the non-human source is selected from at least one of porcine, equine, bovine, caprine or ovine.

6. The reference control composition of any of the preceding claims, wherein the non-human source is selected from one or more of boar, ram, stallion or bull.

7. The reference control composition of any of the preceding claims, wherein the concentration of the agent is from about 0.01 % to about 2.5%.

8. The reference control composition of any of the preceding claims, wherein the agent includes one or more ingredients having one or any combination of the following:
i. a pH of about 7 to about 9;
ii. a hydrophilic-lipophilic balance (HLB) value of about 15.5 to about 18.5;
iii. a viscosity of about 0.5 Pa*s (500 cP) to about 0.55 Pa*s (550 cP) at 25°C;
iv. a density of about 1.075 g/mL to 1.100 g/mL at 25°C; and
v. a surface tension of about 39 dynes/cm to about 52 dynes/cm at 25°C.

9. The reference control composition of any of the preceding claims, wherein the agent includes one or more ingredients having one or any combination of the following:
i. a pH of about 5 to about 7.5;
ii. a hydrophilic-lipophilic balance (HLB) value of about 24 to about 28;
iii. a viscosity of about 1 Pa*s (1000 cP) to about 2.3 Pa*s (2300 cP) at 77°C; and
iv. a surface tension of about 48 dynes/cm to about 50 dynes/cm at 25°C.

10. The reference control composition of any of the preceding claims, wherein the agent includes one or more ingredients having one or any combination of the following:
i. a pH of about 5 to about 9;
ii. a hydrophilic-lipophilic balance (HLB) value of about 15.5 to about 28; and
iii. a surface tension of about 39 dynes/cm to about 52 dynes/cm at 25°C.

11. The reference control composition of any of the preceding claims, wherein the agent includes one or more octylphenol ethoxylates, one or more block copolymers, at least one hydrocarbon chain with one or more ether groups, one or more alcohol groups, a molecule having a hydrophilic portion and a hydrophobic portion, an alkyl ester and/or an alkoxylated ester, an alkyl ether and/or an alkoxylated ester, nonoxynol, alkylphenol ether, a plurality of repeating alkylene oxide units, or any combination thereof..

12. The reference control composition of any of the preceding claims, wherein the pH of the control composition is from about 7 to about 9.5.

13. The reference control composition of any of the preceding claims, wherein the control composition has a closed vial stability of at least about 2 years at a temperature of about 2°C - 10°C.

14. A method for preparing a reference control composition comprising:
obtaining non-human semen containing a plurality of spermatozoa that has been contacted within about thirty minutes of collection with phosphate buffered saline and at least one enzyme; and
adding to the contacted semen a diluent composition comprising one or any combination of a buffer, a viscosity agent, a fixative agent or organic biocide agent and at least one agent for maintaining the plurality of spermatozoa in a substantially aggregate-free condition.

15. A reference control composition comprising:
a. a preserved plurality of non-human spermatozoa, wherein a head portion of each of the plurality of spermatozoa are at least about 25 µM to about 40µM at their largest diameter.
